Europäisches Patentamt

**European Patent Office**

**Office européen des brevets**

(11) Numéro de publication : **0 162 043**
**B1**

(12) **FASCICULE DE BREVET EUROPÉEN**

(45) Date de publication du fascicule du brevet :
13.12.89

(51) Int. Cl.⁴ : **A 61 K 9/46, B 01 J 2/00**

(21) Numéro de dépôt : **84900064.1**

(22) Date de dépôt : **20.12.83**

(86) Numéro de dépôt international :
**PCT/FR 83/00253**

(87) Numéro de publication internationale :
**WO/8402468 (05.07.84 Gazette 84/16)**

(54) **PROCEDE ET APPAREILLAGES DE FABRICATION DE GRANULES ET COMPRIMES EFFERVESCENTS.**

(30) Priorité : 21.12.82 FR 8221476
30.11.83 FR 8319143

(43) Date de publication de la demande :
27.11.85 Bulletin 85/48

(45) Mention de la délivrance du brevet :
13.12.89 Bulletin 89/50

(84) Etats contractants désignés :
BE

(56) Documents cités :
EP–A– 0 076 340
AT–A– 372 299
FR–A– 2 092 893
US–A– 2 877 159
US–A– 2 985 562
US–A– 2 999 293
US–A– 3 401 216
US–A– 3 946 996

(73) Titulaire : **BRU, Jean**
**24 Avenue Raphael**
**F-75016 Paris (FR)**

(72) Inventeur : **BRU, Jean**
**24 Avenue Raphael**
**F-75016 Paris (FR)**

(74) Mandataire : **Portal, Gérard et al**
**Cabinet Beau de Loménie 55, rue d'Amsterdam**
**F-75008 Paris (FR)**

Jouve, 18, rue St-Denis, 75001 Paris, France

## Description

La présente invention concerne un procédé perfectionné de fabrication de mélanges et comprimés effervescents contenant notamment des principes actifs à usage pharmaceutique.

Dans ce domaine, un progrès décisif a été réalisé par les techniques décrites dans les brevets français n° 71 12175 et n° 71 35069.

Ces techniques font intervenir trois stades opératoires :

1) humidification ménagée du bicarbonate de sodium par une très faible quantité d'eau déminéralisée, puis addition de l'acide citrique et éventuellement de glycocolle (liant), le tout dans un mélangeur du type pétrin, ce qui amorce la réaction du bicarbonate sur l'acide citrique ;

2) préséchage du mélange en lit fluidisé obtenu par soufflage d'air chaud, ce qui bloque la réaction ;

3) séchage final également en lit fluidisé obtenu par soufflage d'air chaud.

Les deux brevets précités décrivent avec précision les détails du mode opératoire pour chacune des trois phases : durée, température de l'air, teneur en humidité, vitesse du jet d'air, etc., et l'homme du métier pourra utilement s'y référer.

Bien que très intéressante, cette technique présente l'inconvénient de nécessiter le transfert de la charge, après le stade 1), du mélangeur vers le sécheur. En conséquence, la réaction effervescente déclenchée dans le mélangeur ne peut pas être maîtrisée avec une précision totale car son blocage, qui intervient au stade 2) dans le sécheur, dépend du temps de vidange et de transfert de la charge vers le sécheur, temps qui varie d'un lot au suivant.

Cette variation de temps a une répercussion très importante sur la qualité du grain en fin de granulation.

Ce problème de mise en œuvre industrielle n'a pu être résolu depuis l'invention de la technique, il y a donc environ douze ans, malgré l'intérêt évident qu'il y aurait eu à le résoudre et les tentatives effectuées.

En raison de la réactivité des composés des mélanges effervescents, principalement le bicarbonate et l'acide citrique, dans l'état antérieur de la technique, on a cherché à réaliser une passivation superficielle des composants effervescents en poudre. Par exemple dans le document GERGELY EP-A-76 340, on décrit un procédé de fabrication de comprimés effervescents selon lequel les matières premières en poudre sont traitées dans un dispositif de granulation-séchage comportant des moyens de chauffage, dans lequel on réalise la granulation par ajout d'un solvant de granulation pour former des granulés par mouillage, et le séchage des granulés sous vide, qui sont ensuite comprimés en comprimés. De même, on décrit un appareil de fabrication de comprimés effervescents comprenant un dispositif de granulation-séchage, dans lequel sont traitées des matières premières pour leur granulation à l'aide d'un solvant de granulation, ledit dispositif de séchage comprenant des moyens de chauffage et des moyens de mise sous vide pour réaliser un séchage sous vide. Des moyens de compression sont ensuite prévus pour comprimer en comprimés.

Selon le procédé et l'appareil décrits dans ce document, on réalise une passivation superficielle des composants effervescents en poudre par addition sous vide d'une quantité dosée de solvant polaire de mouillage-granulation et on procède à un contrôle de l'évolution de la pression à l'intérieur du dispositif de granulation-séchage pour soutirer le $CO_2$ libéré par l'ajout de la quantité dosée de solvant polaire pour ramener la pression dans ce dispositif à la pression initiale, puis on recommence avec un nouvel ajout de solvant. Cette technique est très complexe, nécessite un temps important pour aboutir à une passivation superficielle satisfaisante des composés effervescents. Surtout, cette méthode aboutit à une perte importante des capacités effervescentes des comprimés obtenus.

On a maintenant découvert selon l'invention qu'il était possible de mettre en œuvre toutes les réactions et opérations décrites ci-dessus dans un même appareil multi-fonctions, dans lequel la granulation a été réalisée à la pression atmosphérique.

Compte tenu de la très grande précision exigée dans ces opérations pour satisfaire aux exigences très strictes de qualité de l'industrie pharmaceutique, (notamment en ce qui concerne l'homogénéité du granule fini, et l'interruption à un degré d'avancement très précis de la réaction initiée par l'apport de solvant), on ne pensait pas cela possible.

La demanderesse est cependant parvenue à ce résultat, et, de plus, est ainsi parvenue à encore améliorer la qualité des granulés.

Ainsi, la présente invention fournit un procédé de fabrication de comprimés effervescents selon lequel les matières premières en poudre sont traitées dans un dispositif de granulation-séchage comportant des moyens de chauffage, dans lequel on réalise la granulation par ajout d'un solvant de granulation pour former des granulés par mouillage, et le séchage des granulés sous vide, qui sont ensuite comprimés en comprimés, caractérisé en ce qu'on prévoit un appareil disposé verticalement pour former une tour de travail hors poussière, dans laquelle les matières premières sont stockées vers le sommet dans des trémies, qui sont alimentées par gravité dans ledit dispositif de granulation-séchage ; les matières premières sont mélangées dans le dispositif de granulation-séchage puis on introduit ledit solvant de granulation dans ce dispositif de granulation-séchage, on procède à une agitation de l'ensemble des matières premières et du solvant de granulation à la pression atmosphérique jusqu'à obtention d'une granulation, puis on réalise ledit séchage sous vide des granulés ; on refroidit les matières premières séchées et enfin les matières premières séchées refroidies sont alimentées par gravité à un réservoir de stockage.

Selon une caractéristique avantageuse, le solvant de granulation est un solvant choisi parmi le groupe consistant en de l'eau déminéralisée, et une solution aqueuse de dioctylsulfosuccinate de sodium.

Selon une variante de réalisation du procédé, après séchage et refroidissement, les matières premières séchées refroidies sont alimentées par gravité à un dispositif de calibrage. Avantageusement, après calibrage, les matières premières séchées, refroidies, calibrées sont alimentées par gravité dans un mélangeur à ruban.

Selon une autre variante de réalisation, on pèse les matières premières avant tout traitement.

Selon encore une autre variante de réalisation du procédé selon l'invention, l'ensemble des opérations du procédé est automatisé.

D'autres caractéristiques du procédé selon l'invention font l'objet de sous-revendications particulières.

L'invention fournit également un appareil de fabrication de comprimés effervescents comprenant un dispositif de granulation-séchage, dans lequel sont traitées des matières premières pour leur granulation à l'aide d'un solvant de granulation, ledit dispositif de séchage comprenant des moyens de chauffage et des moyens de mise sous vide pour réaliser un séchage sous vide, et des moyens de compression pour les comprimer en comprimés, caractérisé en ce qu'il est disposé verticalement pour former une tour de travail hors poussière, comprenant à l'intérieur, vers le sommet, des trémies, dans lesquelles sont stockées les matières premières, capables d'alimenter par gravité ledit dispositif de granulation-séchage, qui comprend des moyens de mise à la pression atmosphérique au moins pendant ladite granulation et des moyens de séchage sous vide des granulés, ainsi que des moyens d'évacuation par gravité des matières séchées pour les envoyer par gravité dans un refroidisseur ; et au moins un réservoir de stockage recevant par gravité les matières premières séchées refroidies.

Selon une caractéristique avantageuse de cet appareil, celui-ci est caractérisé en ce que le dispositif de séchage comprend des moyens de mélange comprenant un système d'agitation mécanique avec des couteaux destructeurs de mottes, des moyens d'introduction de solvant de granulation comprenant un système d'aspiration de ¡solvant de granulation par création d'un vide ou un système de pulvérisation alimenté par une pompe, des moyens de régulation de la température comprenant une double paroi, dans laquelle circule un fluide caloporteur et un appareillage de vide.

D'autres caractéristiques avantageuses font l'objet de sous-revendications de l'appareil.

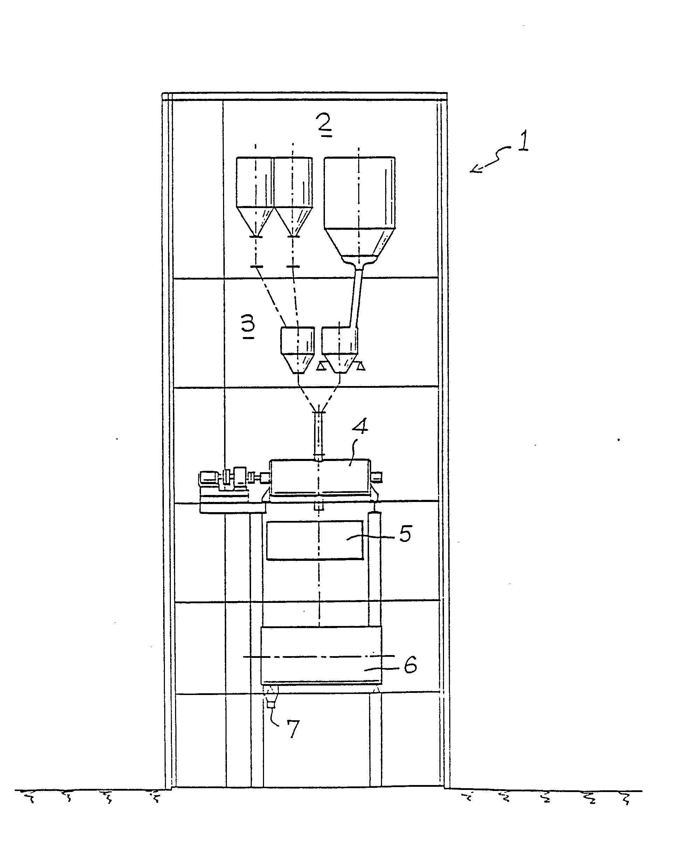

D'autres caractéristiques et avantages de l'invention seront mieux compris à la lecture de la description qui va suivre, et en se référant à la figure unique annexée, sur laquelle on a représenté schématiquement la disposition de l'appareil selon l'invention dans une tour de fabrication verticale.

Dans la figure, les références ont les significations suivantes :

1. tour de fabrication
2. trémies de stockage
3. alimentation automatique
4. appareil de mélange-granulation-séchage
5. trémie d'échange thermique
6. mélangeur à ruban
7. vidange.

Selon l'invention, on a maintenant découvert que, de manière surprenante, les appareils granulateurs-sécheurs dans lesquels la granulation a été réalisée à la pression atmosphérique permettent de réaliser le mélange, la granulation et le séchage dans un seul et même appareil avec les avantages ci-dessous très importants aussi bien en ce qui concerne la qualité des produits que la rentabilité industrielle :

pas de vidange ni de transfert du mélange réactionnel, donc possibilité de stopper la réaction effervescente d'une manière extrêmement précise ; ceci entraîne à son tour la disparition des différences que l'on pouvait noter d'un lot à un autre dans la technique antérieure ;

gain de temps ;

les mélangeurs classiques devaient être placés sous aspiration pour éviter une montée en pression due à la formation de $CO_2$. Ils devaient en outre être décolmatés régulièrement. Ces deux facteurs provoquaient des pertes importantes de poudre, qui ne se retrouvent pas dans le nouveau procédé selon l'invention (environ 1 %) ;

possibilité de paramétrer et automatiser les granulateurs-sécheurs utilisés, ce qui était très difficile sinon impossible dans le cadre de la technique antérieure.

Le principe d'une granulation humide utilisé permet, à partir de poudres de calibre défini, d'obtenir un produit élaboré dont les caractéristiques granulométriques sont adaptées à une compression ultérieure.

Le procédé de granulation selon l'invention permet aussi d'obtenir des mélanges de poudres de composition chimique homogène.

La granulation consiste en une fixation par liaison chimique des particules de bicarbonate de sodium et d'acide citrique. Ces liaisons chimiques sont obtenues par un apport d'une quantité d'eau déminéralisée définie qui provoque une réaction effervescente partielle avec formation de citrate mono-, di-, ou trisodique. Ces molécules de citrate de sodium sont considérées comme des ponts liant les particules de bicarbonate de sodium et les particules d'acide citrique et donnent au mélange des propriétés physiques de compressibilité.

La réaction effervescente déclenchée par un apport externe de 1 à 6 % en poids de solvant aqueux (eau ou solution de mouillant) va se poursuivre d'elle-même car elle est génératrice d'eau, et elle sera bloquée par le séchage sous vide qui suit la granulation.

Le procédé de granulation-séchage est applicable notamment pour la réalisation d'un mélange effervescent type excipient sur lequel on ajoute dans une opération ultérieure les principes actifs et lubrifiants ; à titre d'exemple, nous citerons les paramètres définis pour l'aspirine effervescente tamponnée, pour laquelle on effectue une granulation par voie humide sur trois composés : bicarbonate de sodium, acide citrique et éventuellement glycocolle (liant).

Il est applicable également pour la réalisation d'un mélange effervescent contenant un ou plusieurs principes actifs. A titre d'exemple, nous citerons les paramètres réalisés pour une formule contenant du paracétamol.

L'invention permet de mettre en œuvre, de manière particulièrement efficace, un procédé de fabrication utilisant le vide pour le séchage. Cette technique permet :

d'éliminer la phase de mouillage et de séchage séparés

de raccourcir le temps de fabrication améliorant ainsi la productivité

d'économiser de l'énergie sans oublier le moindre risque de déstabilisation chimique par un traitement à partir de solvants.

Une autre originalité de l'invention réside dans le fait que les appareils ont été groupés dans une tour de fabrication verticale.

Selon cette variante de réalisation, objet de la figure unique annexée, le procédé de fabrication du granulé effervescent destiné à la compression se caractérise par la création d'une tour 1, dans laquelle sont effectuées les opérations suivantes :

1. les pesées de matières premières à partir de trémies de stockage 2,

2. l'alimentation automatique 3 de tout ou partie de ces matières premières dans le mélangeur-granulateur-sécheur 4,

3. le mélange ; la granulation ; et le séchage du granulé effervescent sous vide,

4. le refroidissement dans une trémie d'échange thermique 5 par fluidisation plus compacte qu'un sécheur à lit d'air fluidisé,

5. le calibrage par granulateur oscillant et le mélange final dans un mélangeur à ruban 6 par exemple du type Gondard sur pesons pour calcul du rendement,

6. la vidange 7 de ce mélangeur dans des containers de type Flo-Bin.

Les opérations de 1 à 6 se déroulent verticalement de haut en bas, les transferts ayant lieu par gravité et hors poussière. L'intérêt réside dans le fait que les trois opérations du point 3 (mélange, granulation, séchage) sont automatisables sur le plan de la surveillance du procédé.

De même, les opérations de 1 à 5 dans leur enchaînement chronologique peuvent être automatisées. Cette automatisation rend le procédé économique sur le plan de la main-d'œuvre (1 t/h environ pour deux personnes) par suppression des ruptures de charge entre les opérations.

L'appareil servant au mélange-granulation-séchage est composé d'une cuve parfaitement étanche, munie d'un système d'agitation mécanique destiné au mélange des poudres incorporées et équipée de couteaux démotteurs qui assurent la division des agglomérats formés par le solvant au cours du mouillage. Cette cuve doit également comporter :

une double paroi permettant le passage d'un fluide caloporteur

une trappe d'admission et de vidange des matières premières

un appareillage de vide qui permet d'obtenir une pression minimale de 70 millibars.

Tous les éléments mécaniques ou physiques intervenant dans les opérations successives de granulation-séchage sont contrôlés par sonde thermométrique, par mesure d'ampérage et de vide.

A titre d'exemple, un appareil du type DVT Lödige adapté convient. Une autre originalité de l'invention réside dans l'emploi d'une trémie d'échange thermique pour le refroidissement des granulés.

Les exemples suivants illustrent l'invention sans toutefois en limiter la portée. Pour le mode opératoire général, on se reportera aux brevets français précités. Les exemples 1 à 4 et 6 concernent des compositions qui peuvent être utilisées pour la mise en œuvre du procédé selon l'invention, comme décrit à l'exemple 5.

## Exemple 1

Principe actif : aspirine
Mélange effervescent : bicarbonate de sodium
                                   acide citrique
                                   glycocolle (éventuellement)
pour la fabrication de 255,22 kg de poudres.

## Exemple 2

Granulation sur granulateur-sécheur d'un mélange effervescent contenant plusieurs principes actifs :

Formule de granulation

| | |
|---|---:|
| paracétamol | 28 kg |
| carbonate monosodique | 100 kg |
| carbonate monopotassique | 3,740 kg |
| sorbitol | 25,500 kg |
| acide citrique anhydre | 73,000 kg |
| acide ascorbique | 17,000 kg |
| Total | 247,24 kg |

## Exemple 3

| | |
|---|---:|
| sulfate disodique | 20,200 kg |
| bromure de sodium | 7,100 kg |
| phosphate disodique | 13,800 kg |
| carbonate monosodique | 101,000 kg |
| acide citrique anhydre | 89,000 kg |

Nature du solvant : solution aqueuse de succinate de doxylamine.

## Exemple 4

| | |
|---|---:|
| carbonate monosodique | 108,000 kg |
| acide citrique anhydre | 14,500 kg |
| citrate de bétaïne | 142,000 kg |

Nature du solvant : eau déminéralisée.

## Exemple 5

Formulation

paracétamol
vitamine C
bicarbonate de sodium
acide citrique
bicarbonate de potassium
sorbitol

Les essais ont été effectués sur un mélange de 58 kg de poudres.

Opération 1 : Prémélange

Les différentes matières premières précitées sont incorporées successivement dans le mélangeur 4 sans tenir compte des incompatibilités physiques qui peuvent exister entre elles. Le prémélange s'effectue en 3 minutes. Pendant ce laps de temps, on procède à une élévation de température de la poudre par l'introduction d'un liquide caloporteur dans la double enveloppe du mélangeur.

Opération 2 : Incorporation du solvant dans le mélangeur

Le solvant est un mélange eau/dioctylsulfosuccinate de sodium.
Mouillage par pulvérisation ou aspiration du solvant sous vide partiel.
Concentration du solvant : 0,64 % par rapport au poids de la poudre.

Opération 3 : Granulation

La granulation est effectuée sous la pression atmosphérique durant 5 minutes.

Opération 4 : Séchage

Il est obtenu par la diminution de la pression atmosphérique jusqu'à 70 millibars ; à cette pression, le point d'ébullition du solvant est atteint. Le séchage se poursuit en élevant la température.

Cette opération dure environ 37 minutes, l'arrêt du séchage étant déclenché par contrôle de l'humidité résiduelle de la poudre.

Les avantages de cette technique sont :

l'obtention d'un granulé de meilleure qualité, notamment plus homogène,

l'utilisation de zones thermiques suffisamment basses pour que les produits actifs thermolabiles ne soient pas déstabilisés,

l'automatisation complète possible,

l'apport calorifique nécessaire à l'évaporation du solvant est très faible.

Exemple 6 : Exemple de mélange par compression directe

Formulation

carbasalate calcique
carbamate de lysine
acide citrique
arôme
PEG (polyéthylèneglycol)
saccharinate d'ammonium

Description de la fabrication

1. Le carbasalate calcique sera stocké dans l'une des trémies 2. Il sera ensuite prépesé en 3 et incorporé dans le mélangeur-granulateur-sécheur 4.

Dans cette variante de l'invention, l'automatisme de la tour permet d'annuler les fonctions mélange et granulation et de ne conserver que la fonction séchage de l'appareil 4.

2. Après séchage, le carbasalate calcique est envoyé dans le refroidisseur 5 afin de ramener le produit à sa température initiale.

3. Le calibrage est effectué, soit par simple passage sur grille, soit par broyage, le choix devant se faire en fonction de la nature des produits qui nous sont livrés.

4. Vidange dans un mélangeur à ruban 6, dans lequel on fait la pesée exacte du produit.

Les quatre opérations précitées sont ensuite répétées pour le carbamate de lysine et l'acide citrique.

Après réception de ces trois composants dans le mélangeur, on effectue dans le mélangeur à ruban l'apport des composants annexes : arôme, PEG et saccharinate d'ammonium.

La mise en route du mélangeur et son principe de fonctionnement permettent d'assurer la qualité exigée sur le plan homogénéité du principe actif de cette formule.

**Revendications**

1. Procédé de fabrication de comprimés effervescents selon lequel les matières premières en poudre sont traitées dans un dispositif de granulation-séchage comportant des moyens de chauffage, dans lequel on réalise la granulation par ajout d'un solvant de granulation pour former des granulés par mouillage, et le séchage des granulés sous vide, qui sont ensuite comprimés en comprimés, caractérisé en ce qu'on prévoit un appareil disposé verticalement pour former une tour de travail hors poussière, dans laquelle les matières premières sont stockées vers le sommet dans des trémies, puis sont alimentées par gravité dans ledit dispositif de granulation-séchage ; les matières premières sont mélangées dans le dispositif de granulation-séchage puis on introduit ledit solvant de granulation dans ce dispositif de granulation-séchage, on procède à une agitation de l'ensemble des matières premières et du solvant de granulation à la pression atmosphérique jusqu'à obtention d'une granulation, puis on réalise ledit séchage sous vide des granulés, on refroidit les matières premières séchées et enfin les matières premières séchées refroidies sont alimentées par gravité à un réservoir de stockage.

2. Procédé selon la revendication 1, caractérisé en ce que le solvant de granulation est un solvant choisi parmi le groupe consistant en de l'eau déminéralisée, et d'une solution aqueuse de dioctylsulfosuccinate de sodium.

3. Procédé selon l'une des revendications 1 ou 2, caractérisé en ce que, après séchage et refroidissement, les matières premières séchées et refroidies sont alimentées par gravité à un dispositif de calibrage.

4. Procédé selon la revendication 3, caractérisé en ce que, après calibrage, les matières premières séchées, refroidies, calibrées sont alimentées par gravité dans un mélangeur à ruban.

5. Procédé selon l'une quelconque des revendications 1 à 4, caractérisé en ce qu'on pèse les matières premières avant tout traitement.

6. Procédé selon la revendication 5, caractérisé en ce que l'ensemble des opérations du procédé est automatisé.

7. Procédé selon l'une quelconque des revendications 1 à 6, caractérisé en ce que le dispositif de séchage précité comprend des moyens de mélange comprenant un système d'agitation mécanique avec des couteaux de destruction de mottes, des moyens d'introduction de solvant de granulation, comprenant un système d'aspiration du solvant de granulation par création d'un vide ou un système de pulvérisation alimenté par une pompe, des moyens de régulation de température comprenant une double paroi où circule un fluide caloporteur, et des moyens de création d'un vide.

8. Procédé selon l'une quelconque des revendications 1 à 7, caractérisé en ce que les matières premières sont du bicarbonate de sodium, de l'acide citrique et le solvant de granulation est de l'eau déminéralisée.

9. Procédé selon la revendication 8, caractérisé en ce que les matières premières comprennent également du glycocolle.

10. Procédé selon l'une quelconque des revendications 1 à 7, caractérisé en ce que les matières premières comprennent :

    du paracétamol

    du carbonate monosodique

    du carbonate monopotassique

    du sorbitol

    de l'acide citrique anhydre

    de l'acide ascorbique

et le solvant de granulation est une solution aqueuse de dioctylsulfosuccinate de sodium.

11. Procédé selon l'une quelconque des revendications 1 à 7, caractérisé en ce que les matières premières comprennent :

    du carbasalate de calcium

    du carbamate de lysine

    et de l'acide citrique.

12. Procédé selon l'une quelconque des revendications 1 à 7, caractérisé en ce que les matières premières comprennent :

    du carbasalate de calcium

    du carbamate de lysine

    de l'acide citrique

    un arôme

    du PEG (polyéthylèneglycol)

    du saccharinate d'ammonium.

13. Appareil de fabrication de comprimés effervescents comprenant un dispositif de granulation-séchage, dans lequel sont traitées des matières premières pour leur granulation à l'aide d'un solvant de granulation, ledit dispositif de séchage comprenant des moyens de chauffage et des moyens de mise sous vide pour réaliser un séchage sous vide, et des moyens de compression pour les comprimer en comprimés, caractérisé en ce qu'il est disposé verticalement pour former une tour de travail hors poussière (1), comprenant à l'intérieur, vers le sommet, des trémies (2) dans lesquelles sont stockées les matières premières capables d'alimenter par gravité ledit dispositif de granulation-séchage (4), qui comprend des moyens de mise à la pression atmosphérique au moins pendant ladite granulation et des moyens de séchage sous vide des granulés ainsi que des moyens d'évacuation par gravité des matières premières séchées pour les envoyer par gravité dans un refroidisseur (5) ; et au moins un réservoir de stockage recevant par gravité les matières premières séchées refroidies.

14. Appareil selon la revendication 13, caractérisé en ce qu'il comprend des moyens de calibrage des matières premières après le refroidisseur (5) précité.

15. Appareil selon la revendication 13 ou 14, caractérisé en ce que, après le calibreur, il comprend un mélangeur à ruban (6).

16. Appareil selon la revendication 15, caractérisé en ce qu'il comprend des moyens de pesage des matières premières avant tout traitement.

17. Appareil selon l'une des revendications 13 à 16, caractérisé en ce que le dispositif de séchage comprend des moyens de mélange comprenant un système d'agitation mécanique avec des couteaux destructeurs de mottes, des moyens d'introduction de solvant de granulation comprenant un système d'aspiration de solvant de granulation par création d'un vide ou un système de pulvérisation alimenté par une pompe, des moyens de régulation de la température comprenant une double paroi dans laquelle circule un fluide caloporteur et un appareillage de vide.

7

**Claims**

1. Process for the preparation of effervescent tablets according to which the powdered raw materials are treated in a granulation-drying device comprising heating means, and consisting in carrying out the granulation by addition of a granulating solvent in order to form granules by wetting, and in drying the granules in vacuo, said granules being thereafter compressed into tablets, characterized in that an apparatus is provided which is mounted vertically in order to form a dust-free working tower, in which the raw materials are stored in hoppers situated near the upper part, and then gravity-fed in said granulation-drying device ; the raw materials are mixed in the granulation-drying device, and then the granulating solvent is introduced in said device, the mixture of raw materials and granulating solvent is then stirred at the atmospheric pressure, until a granulation is obtained, and the granules are dried in vacuo, the dried raw materials are then cooled and finally the cooled dried raw materials are gravity-fed to a storage container.

2. Process according to claim 1, characterized in that the granulating solvent is a solvent selected from the group consisting of demineralised water, and of an aqueous solution of sodium dioctylsulfosuccinate.

3. Process according to one of claims 1 or 2, characterized in that, after drying and cooling, the dried and cooled raw materials are gravity-fed to a calibrating device.

4. Process according to claim 3, characterized in that, after calibrating, the dried, cooled and calibrated raw materials are gravity-fed in a band mixer.

5. Process according to any one of claims 1 to 4, characterized in that the raw materials are weighed before any treatment.

6. Process according to claim 5, characterized in that the totality of the operations of the process is automated.

7. Process according to any one of claims 1 to 6, characterized in that said drying device comprises mixing means equipped with a mechanical stirring system having lump-breaking cutters, means of introducing the granulating solvent, consisting in a granulating solvent suction system by creation of a vacuum or a pump-supplied spraying system, means of controlling the temperature, comprising a double wall in which circulates a heat-exchanging fluid, and means for creating a vacuum.

8. Process according to any one of claims 1 to 7, characterized in that the raw materials are sodium citric acid and the granulating solvent is demineralised water.

9. Process according to claim 8, characterized in that the raw materials further include glycocoll.

10. Process according to any one of claims 1 to 7, characterized in that the raw materials comprise :
paracetamol
monosodium carbonate
monopotassium carbonate
sorbitol
anhydrous citric acid
ascorbic acid
and the granulating solvent is an aqueous solution of sodium dioctyl-sulfosuccinate.

11. Process according to any one of claims 1 to 7, characterized in that the raw materials comprise :
calcium carbasalate
lysine carbamate
and citric acid
flavouring agent.

12. Process according to any one of claims 1 to 7, characterized in that the raw materials comprise :
calcium carbasalate
lysine carbamate
citric acid
a flavouring agent
PEG (polyethyleneglycol)
ammonium saccharinate.

13. Apparatus for the preparation of effervescent tablets comprising a granulation-drying device, in which raw materials are subjected to a granulating treatment with a granulating solvent, said drying device comprising heating means and vacuum-creating means in order to carry out a vacuum-drying, and compression means for compressing the granules into tablets, characterized in that said apparatus is disposed vertically in order to constitute a dust-free tower (1), provided on the inside and near the upper part, with hoppers (2) for storing the raw materials which can be gravity-fed to said granulation-drying device (4), comprising means for bringing the pressure to the atmospheric pressure at least during said granulation and means for vacuum-drying the granules as well as means for gravity-discharging the dried raw materials in order to feed them by gravity to a cooler (5) ; and at least one storage container receiving by gravity the dried and cooled raw materials.

14. Apparatus according to claim 13, characterized in that it comprises means of calibrating the raw materials provided after said cooler (5).

15. Apparatus according to claim 13 or 14, characterized in that a band mixer (6) is provided after the calibrating device.

16. Apparatus according to claim 15, characterized in that it comprises means of weighing the raw materials before any treatment.

17. Apparatus according to one of claims 13 to 16, characterized in that the drying device comprises mixing means equipped with a mechanical stirring system with lump-breaking cutters, means of introducing granulating solvent which comprise a suction system for the granulating solvent by creation of a vacuum, or a pump-fed spraying system, means for controlling the temperature comprising a double wall in which circulates a heat-exchanging fluid and means for creating a vacuum.


**Patentansprüche**

1. Verfahren zur Herstellung von Brause-Tabletten, wonach die pulverförmigen Rohmaterialien in einer Granulier-Trockeneinrichtung, die Mittel zum Heizen aufweist, behandelt werden, in welcher die Granulation durch Zugabe eines Granulier-Lösungsmittels zum Granulieren erfolgt, um das Granulat durch Befeuchten zu bilden, und die Trocknung des Granulats unter Vakuum erfolgt, welches anschließend zu Tabletten zusammengepreßt wird, dadurch gekennzeichnet, daß ein vertikal angeordneter Apparat vorgesehen wird, der einen Arbeitsturm außerhalb von Staub bildet, in welchem die Rohmaterialien nahe der obersten Stelle in Fülltrichtern gespeichert sind, dann durch Schwerkraft in die Granulier-Trockeneinrichtung eingespeist werden ; daß die Rohmaterialien in der Granulier-Trockeneinrichtung gemischt werden, worauf das Granulier-Lösungsmittel in diese Granulier-Trockeinrichtung eingespeist wird, daß das Gemisch der Rohmaterialien und des Lösungsmittels bei atmosphärischem Druck bis zur Bildung eines Granulats durchmengt wird, daß daraufhin die Trocknung des Granulats unter Vakuum erfolgt, die trockenen Rohmaterialien gekühlt werden und schließlich die getrockneten und gekühlten Rohmaterialien durch deren Schwerkraft in einen Vorratsbehälter eingespeist werden.

2. Verfahren nach Anspruch 1, dadurch gekennzeichnet, daß das Granulier-Lösungsmittel aus der Gruppe ausgewählt ist, die entmineralisiertes Wasser und eine wässrige Lösung von Natrium-Dioktylsulfosukinat umfaßt.

3. Verfahren nach Anspruch 1 oder 2, dadurch gekennzeichnet, daß nach der Trocknung und der Kühlung die getrockneten und gekühlten Rohmaterialien durch Schwerkraft in eine Kalibrierungseinrichtung eingespeist werden.

4. Verfahren nach Anspruch 3, dadurch gekennzeichnet, daß nach der Kalibrierung die getrockneten, gekühlten und kalibrierten Rohmaterialien durch Schwerkraft zu einem Bandmischer gefördert werden.

5. Verfahren nach einem der Ansprüche 1 bis 4, dadurch gekennzeichnet, daß die Rohmaterialien vor jedweder Behandlung gewogen werden.

6. Verfahren nach Anspruch 5, dadurch gekennzeichnet, daß das gesamte Herstellungsverfahren automatisiert ist.

7. Verfahren nach einem der Ansprüche 1 bis 6, dadurch gekennzeichnet, daß die Trockeneinrichtung Mittel zum Mischen umfaßt, die eine Einrichtung zum mechanischen Rühren mit Entklumpungsmessern, eine Einrichtung zum Einführen eines Granuliermittels mit einer Ansaugeinrichtung des Granuliermittels durch Schaffung eines Unterdrucks oder eine Sprüheinrichtung, die durch eine Pumpe gespeist wird, umfaßt, Mittel zum Regulieren der Temperatur mit einem Doppelmantel, in dem eine Wärmeaustauschflüssigkeit zirkuliert, und eine Unterdruckeinrichtung zum Erzeugen eines Unterdrucks umfaßt.

8. Verfahren nach einem der Ansprüche 1 bis 7, dadurch gekennzeichnet, daß die Rohmaterialien Natriumbicarbonat, Zitronensäure sind und das Lösungsmittel demineralisiertes Wasser ist.

9. Verfahren nach Anspruch 8, dadurch gekennzeichnet, daß die Rohmaterialien außerdem Glykokol umfassen.

10. Verfahren nach einem der Ansprüche 1 bis 7, dadurch gekennzeichnet, daß die Rohmaterialien umfassen :

« Paracetamol »
Natriumcarbonat
Kaliumcarbonat
Sorbit
wasserfreie Zitronensäure
Askorbinsäure
und daß das Lösungsmittel eine wäßrige Lösung von Natrium-Dioktylsulfosukinat ist.

11. Verfahren nach einem der Ansprüche 1 bis 7, dadurch gekennzeichnet, daß die Rohmaterialien umfassen :

Kalziumcarbasalat
Lysincarbamat
Zitronensäure.

12. Verfahren nach einem der Ansprüche 1 bis 7, dadurch gekennzeichnet, daß die Rohmaterialien umfassen :

Kalziumcarbasalat

Lysincarbamat
Zitronensäure
ein Aroma
Polyäthylenglykol (PEG)
Ammonium-Saccharin.

13. Einrichtung zur Herstellung von Brausetabletten, die eine Granulier-Trockeneinrichtung umfaßt, in welcher Rohmaterialien zum Granulieren mit einem Granuliermittel behandelt werden, wobei die Trockeneinrichtung Mittel zum Heizen und Mittel zur Erzeugung eines Unterdrucks aufweist, um eine Trocknung unter Unterdruck durchzuführen, sowie Mittel zum Zusammenpressen umfaßt, um sie zu Tabletten zusammenzupressen, dadurch gekennzeichnet, daß sie vertikal ausgebildet ist, um einen Arbeitsturm (1) außerhalb von Staub zu bilden, der in seinem Inneren nahe der obersten Stelle Fülltrichter aufweist, in denen die Rohmaterialien gespeichert sind und die dazu eingerichtet sind, durch Schwerkraft die Granulier-Trockeneinrichtung (4) zu speisen, welche Mittel zum Einstellen auf Atmosphärendruck wenigstens während des Granulierens aufweist, Mittel zum Trocknen des Granulats unter Unterdruck sowie Mittel zum Entleeren der getrockneten Rohmaterialien durch Schwerkraft umfaßt, um diese durch Schwerkraft in einen Kühler (5) einzuspeisen, und daß wenigstens ein Vorratsbehälter vorgesehen ist, der durch Schwerkraft die gekühlten und getrockneten Rohmaterialien aufnimmt.

14. Einrichtung nach Anspruch 13, dadurch gekennzeichnet, daß sie eine Kalibriereinrichtung zum Kalibrieren der Rohmaterialien in Flußrichtung hinter dem Kühler (5) aufweist.

15. Einrichtung nach Anspruch 13 oder 14, dadurch gekennzeichnet, daß sie in Flußrichtung hinter der Kalibriereinrichtung einen Bandmischer (6) umfaßt.

16. Einrichtung nach Anspruch 15, dadurch gekennzeichnet, daß sie eine Wägeeinrichtung zum Wiegen der Rohmaterialien vor jeder Behandlung umfaßt.

17. Einrichtung nach einem der Ansprüche 13 bis 16, dadurch gekennzeichnet, daß die Trockeneinrichtung eine Mischeinrichtung umfaßt, die eine Einrichtung zum mechanischen Rühren mit Entklumpungsmessern, eine Einrichtung zum Einführen eines Lösungsmittels zum Granulieren mit einer Ansaugeinrichtung des Lösungsmittels zum Granulieren durch Schaffung eines Unterdrucks oder einer Sprüheinrichtung, die durch eine Pumpe gespeist wird, umfaßt, Mittel zum Regulieren der Temperatur mit einem Doppelmantel, in dem eine Wärmeaustauschflüssigkeit zirkuliert, sowie eine Unterdruckeinrichtung umfaßt.